(19) Europäisches Patentamt
European Patent Office
Office européen des brevets

(11) EP 3 821 801 A1

(12) EUROPEAN PATENT APPLICATION
published in accordance with Art. 153(4) EPC

(43) Date of publication:
19.05.2021 Bulletin 2021/20

(51) Int Cl.:
A61B 5/0452 (2006.01)   A61B 5/0402 (2006.01)

(21) Application number: 19835103.3

(22) Date of filing: 28.06.2019

(86) International application number:
PCT/CN2019/093836

(87) International publication number:
WO 2020/011033 (16.01.2020 Gazette 2020/03)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(30) Priority: 12.07.2018 CN 201810765127

(71) Applicant: Shanghai Microport Ep Medtech Co.,
Ltd.
Shanghai 201318 (CN)

(72) Inventors:
• XIA, Yunlong
Shanghai 201318 (CN)

• ZHANG, Qingchun
Shanghai 201318 (CN)
• SHEN, Liuping
Shanghai 201318 (CN)
• WANG, Lu
Shanghai 201318 (CN)
• SUN, Yiyong
Shanghai 201318 (CN)

(74) Representative: Patentship
Patentanwaltsgesellschaft mbH
Elsenheimerstraße 65
80687 München (DE)

(54) DETERMINING DEVICE AND MAPPING SYSTEM FOR ORIGIN OF ARRHYTHMIA

(57) A determination device (100) for determining the origin of an arrhythmia and a mapping system (200) are disclosed. In the determination device (100), an analysis unit (120) is configured to process ECG data extracted over a predetermined period of time by a data extracting unit (110) and input the processed data into a determination model (131), where a calculation is performed thereon to produce origin information about the origin. An output unit (140) is configured to output the origin information, and a model configuration unit (130) is configured to configure the determination model (131). The determination device (100) allows identifying a cardiac site suitable for focused mapping, thereby dispensing with the need to map the whole heart and shortening the time required for mapping. The mapping system (200) includes the determination device (100). With the origin information about the arrhythmia's origin, a physician can know a cardiac site corresponding to the origin of the arrhythmia in a timely manner before or during a procedure. This can reduce the time required for mapping and result in an enhancement in surgical efficiency.

Fig. 1

**Description**

**Technical Field**

The present invention relates to the field of medical devices and, in particular, to a determination device and a mapping system for the origin of an arrhythmia.

**Background**

An arrhythmia may occur as an abnormal rate and/or rhythm of the heartbeat due to the conduction of an abnormal sinus node impulse or an impulse originating outside the sinus node, which is delayed or blocked or takes place in an irregular pathway, i.e., an abnormality in the generation and/or propagation of cardiac electrical activity. Arrhythmias are an important group of cardiovascular diseases and may occur either alone or in company with another cardiovascular disease or other such diseases.

Arrhythmias, such as atrial and ventricular, are typically triggered by abnormalities such as ectopic beats, which occur in certain sites in the heart, and a mapping system can be used to locate such arrhythmogenic sites in the heart and thus allow treatment thereof. However, traditional mapping systems require mapping of the whole heart, which is time-consuming. Some experienced physicians can shorten the time required for mapping by approximately determining a likely cardiac site of the origin of an arrhythmia based on, for example, information from 12-Lead ECG waveforms before or during the procedure and then directly performing focused mapping of this site. However, due to the lack of well-defined and generalized determination standards, many physicians are insufficient in related experience and knowledge, coupled with the fact that learning how to identify meaningful information from 12-Lead ECG waveforms is highly challenging and has hindered its wider application in heart surgery.

Therefore, it is desirable to develop a tool which can reduce the time required for mapping through allowing even a novice physician to obtain the information about the origin of an arrhythmia in a timely manner before or during the procedure and thus providing him/her with an option for direct focused mapping of a determined site of origin of arrhythmia.

**Summary of the Invention**

It is an objective of the present invention to provide a determination device for determining the origin of an arrhythmia and a mapping system, which can help a physician obtain the information about the origin of an arrhythmia in the heart in a timely manner before or during the procedure, thus facilitating direct focused mapping of an associated intracardiac site of the origin.

According to one aspect of the present invention, there is provided a determination device for an origin of an arrhythmia, configured to determine the origin of the arrhythmia, the determination device including: a data extracting unit configured to extract electrocardiogram (ECG) data over a predetermined period of time; an analysis unit configured to process the ECG data and input the processed ECG data into a determination model to perform a calculation to obtain an origin information about the origin of the arrhythmia; a model configuration unit configured to configure the determination model; and an output unit configured to output the origin information.

Optionally, the analysis unit may include: a pre-processing subunit configured to pre-process the ECG data; a locating subunit configured to derive at least one abnormal waveform feature and a value of the abnormal waveform feature from the pre-processed ECG data, the abnormal waveform feature being related to the arrhythmia; and a calculating subunit configured to input the value of the abnormal waveform feature into the determination model to perform a calculation.

Optionally, the locating subunit may be further configured to derive at least one global waveform feature and a value of the global waveform feature from the pre-processed ECG data, the global waveform feature being related to a normal heart rhythm, and wherein the calculating subunit is further configured to input the value of the global waveform feature into the determination model to perform a calculation. Optionally, the global waveform features may include a heart rate, a P-waveform direction, a P-waveform amplitude, a P-waveform duration, a global QRS waveform direction, a global QRS waveform type, a global QRS waveform amplitude, a global QRS waveform duration, a T-waveform direction, a T-waveform amplitude, a T-waveform duration, a global PR interval and a global QTc interval.

Optionally, the value of the global waveform feature may be presented by a global waveform data array.

Optionally, each column of the global waveform data array may correspond to values of one item of the global waveform feature.

Optionally, the ECG data may be extracted from an 8-Lead ECG, 12-Lead ECG or 20-Lead ECG, and wherein each row of the global waveform data array corresponds to a respective lead

Optionally, the calculating subunit may be configured to input an abnormal waveform data array into the determination model, the abnormal waveform data array having elements corresponding to the respective values of the abnormal waveform feature.

Optionally, the abnormal waveform features may include an abnormal QRS waveform direction, an abnormal QRS waveform type, an abnormal QRS waveform amplitude, an abnormal QRS waveform duration, an abnormal PR interval and an abnormal QTc interval.

Optionally, each column of the abnormal waveform data array may correspond to values of one item of the abnormal waveform feature.

Optionally, the ECG data may be extracted from an 8-Lead ECG, 12-Lead ECG or 20-Lead ECG, and wherein each row of the abnormal waveform data array corresponds to a respective lead.

Optionally, the determination model may include a plurality of origin categories, each associated with one or more determination indicators for making determinations on the ECG data that is input into the determination model, wherein the origin belongs to at least one of the plurality of origin categories.

Optionally, the plurality of origin categories may include "Left Ventricle", "Inferior Anterior Wall of Right Ventricle", "Inferior Posterior Wall of Right Ventricle", "Bundle of His", "Anterior Part of Free Wall of Right Ventricular Outflow Tract", "Posterior Part of Free Wall of Right Ventricular Outflow Tract", "Middle Part of Free Wall of Right Ventricular Outflow Tract", "Anterior Part of Septal Wall of Right Ventricular Outflow Tract", "Posterior Part of Septal Wall of Right Ventricular Outflow Tract" and "Middle Part of Septal Wall of Right Ventricular Outflow Tract".

Optionally, the determination model may be a feature classification model configured to classify the ECG data that is input into the determination model using a plurality of trigger instructions and the determination indicators, wherein when the ECG data that is input into the feature classification model satisfies all the determination indicators associated with a specific origin category of the plurality of origin categories, the origin is determined as belonging to the specific origin category and the origin information is output as an information associated with the specific origin category.

Optionally, the determination model may be a probability comparison model configured with a plurality of weighting functions representing probabilities of the arrhythmia for each of the plurality of origin categories, wherein a calculation is performed by substituting the ECG data input to the probability comparison model into the plurality of weighting functions to obtain one or more of the plurality of origin categories with a highest probability, and wherein the origin is determined as belonging to the one or more of the plurality of origin categories with the highest probability, and the origin information is output as information associated with the one or more of the plurality of origin categories with the highest probability.

Optionally, each of the plurality of weighting functions comprises the sum of the products of values of the determination indicators associated with the plurality of origin categories and respective weight coefficients, each of the weight coefficients reflecting a weight of the multiplied value of the determination indicator in the plurality of weighting functions.

Optionally, the weight coefficients may be obtained from paired comparisons.

Optionally, the determination model may be a neural network classification model configured to train a neural network to derive a functional relationship between a set of ECG data and a set of the plurality of origin categories and to test and classify the ECG data input to the neural network classification model, and wherein the origin belongs to one of the plurality of origin categories identified from the classification and the origin information is output as an information associated with the origin category identified from the classification.

Optionally, the output unit may be configured to output the origin information to a terminal, and the origin information is marked on a heart model displayed on the terminal.

Optionally, the heart model may be a standard heart model, a modeled heart model or a CT-matched heart model.

Optionally, the arrhythmia may be a ventricular premature beat.

According to another aspect of the present invention, there is provided a mapping system for mapping a patient's heart, the mapping system including: an electrocardiogram (ECG) acquisition module for capturing ECG data of the patient; and a mapping module for determining an origin of an arrhythmia and mapping the patient's heart, wherein the mapping module includes the determination device as defined above.

Optionally, the mapping system may further include a display module for displaying an origin information about the origin; and/or a CT image processing module for identifying a CT image of the patient's heart and registering the CT image with a standard or modeled heart model to establish a CT-matched heart model.

In the provided determination device, the analysis unit is configured to process ECG data extracted over a predetermined period of time and input the processed data to the determination model, where a calculation is performed thereon to produce the origin information about the origin. With the origin information, a physician can know a cardiac site suitable for focused mapping, thereby dispensing with the need to map the whole heart and shortening the time required for mapping. The determination model used by the determination device determines the origin of the arrhythmia in a general manner, which is conducive to the standardization and promotion of cardiac mapping technology.

In the provided mapping system, the mapping module includes the determination device that can provide information about the origin of an arrhythmia. Therefore, with the mapping system, a physician can know a cardiac site suitable for focused mapping in a timely manner before or during a procedure. This reduces the time required for mapping and results in enhanced surgical efficiency.

The provided determination device may include a software program, which is stored in a computer readable medium to determine the origin of an arrhythmia during the treatment of the arrhythmia. The obtained information about the origin can help a physician know a cardiac site suitable for focused mapping before the heart is mapped, thus shortening the time required for mapping.

The provided determination device may also include a programmed computer system with such a software program, which can, when executed, provide information about the origin of an arrhythmia.

**Brief Description of The Drawings**

Fig. 1 is a schematic illustration of a determination device for determining the origin of an arrhythmia according to embodiments of the present invention.

Fig. 2 is a flow chart illustrating how an analysis unit operates in accordance with an embodiment of the present invention.

Fig. 3 is a schematic illustration of a feature classification model according to an embodiment of the present invention.

Fig. 4 is a schematic illustration of a probability comparison model according to an embodiment of the present invention.

Fig. 5 is a schematic illustration of a neural network classification model according to an embodiment of the present invention.

Fig. 6 schematically illustrates a mapping system according to embodiments of the present invention.

Fig. 7 is a schematic illustration of a mapping process performed by the mapping system according to an embodiment of the present invention.

In these figures, 100-determination device; 110-data extracting unit; 120-analysis unit; 121-pre-processing subunit; 122-locating subunit; 123-calculating subunit; 130-model configuration unit; 131-determination model; 140-output unit; 101-data buffer; 200-mapping system; 210-ECG acquisition module; 211-ECG lead; 220-mapping module; 221-locating device; 222-mapping catheter; 230-display module; 240-CT image processing module.

**Detailed Description of Preferred Embodiments**

Specific embodiments of the present invention will be described in greater detail with reference to the accompanying drawings. Features and advantages of the invention will be more apparent from the following detailed description. Note that the figures are provided in a very simplified form not necessarily presented to scale, with their only intention to enhance convenience and clarity in explaining the disclosed embodiments. Furthermore, the terminology as used herein is for the purpose of describing the particular embodiments only and is not intended to be limiting of the invention. In this specification, singular references include the plural, unless the context clearly dictates otherwise. It will be further understood that the terms "comprises" and/or "comprising," when used in this specification, specify the presence of stated compounds, ingredients, components, steps, operations, and/or elements, but do not preclude the presence or addition of one or more other compounds, ingredients, components, steps, operations, and/or elements thereof.

In addition, components in the present invention, such as logically separable software (computer programs), hardware or equivalents thereof, are generally referred to as "units" or "modules". Therefore, "units" mentioned in the embodiments herein include both those in computer programs and those in hardware settings. Therefore, embodiments disclosed herein also include computer programs including instructions, which may be programs for carrying out each of steps in a computer, programs for implementing each computer function as a tool, or programs for causing a computer to perform each of functions, systems or methods. "Units" or "modules" are activated by such computer programs. While the terms "storing", "stored" or equivalents thereof can be used herein for ease of description, they are meant to describe the storage of programs or the storage of computer programs in storage member in a controlled manner. Although individual "modules" and "units" can be configured in substantial one-to-one correspondence with respective functions, in practice, a single module may be configured to have one or more programs, or multiple modules may be configured to have either a single program or multiple programs. In a distributed or parallel environment, multiple modules may be implemented by a single computer, or a single module may be implemented by multiple computers. A single module may include additional modules.

Herein, an "device" and/or "system" may include multiple computers, hardware elements, devices or the like that are interconnected by communication appliances in an injection network with one-to-one connectivity, or a single computer, hardware element, device or the like capable of implementing a process in accordance with the present invention.

Further, each process, whether performed by a single module or unit, or by multiple modules or units, may involve reading required information from storage member such a memory device and writing the results from the process into the storage member. Therefore, for each process described herein, a description of reading from and writing into storage member prior and subsequent to the process may be omitted. Herein, "storage member" may include hard drive disks, random access memory (RAM) devices, external storage media, storage member accessible via communication connections, registers in central processing units (CPUs), etc.

In embodiments of the present invention, there is provided a determination device for determining the origin of an arrhythmia, in particular for extracting and analyzing ECG data over a predetermined period of time and outputting information about the origin. Here, the term "origin" refers to an associated site in the heart where the arrhythmia originates, and "origin information" refers to information reflecting the origin determination results, which may be in the form of any or a combination of images, text and voice.

According to embodiments of the present invention, the ECG data may include data from electrocardiograms (ECGs). An ECG can be obtained from multiple lead electrodes, which are attached to the surface of a patient's body to record, and provide spatial information about, the heart's electrical activity. Taking a 12-Lead ECG as an example, the 12-Lead ECG records cardiac electrical activity with 12 leads, each for a respective channel, specifically, the 12-Lead ECG includes the 12 leads, which are denoted as Lead I, Lead II, Lead III, Lead aVR, Lead aVL, Lead aVF, Lead V1, Lead V2, Lead V3, Lead V4, Lead V5 and Lead V6. The 12 leads represent the leads located on respective body parts. The ECG data may include, for example, information of ECG waveforms such as their type, direction, amplitude, duration and interval. However, without being limited to those, the ECG data may also be other various arrhythmia-related cardiac electrical activity data. Those skilled in the art can know measurement acquisition of ECG data from published information, and any further description thereof will be omitted here.

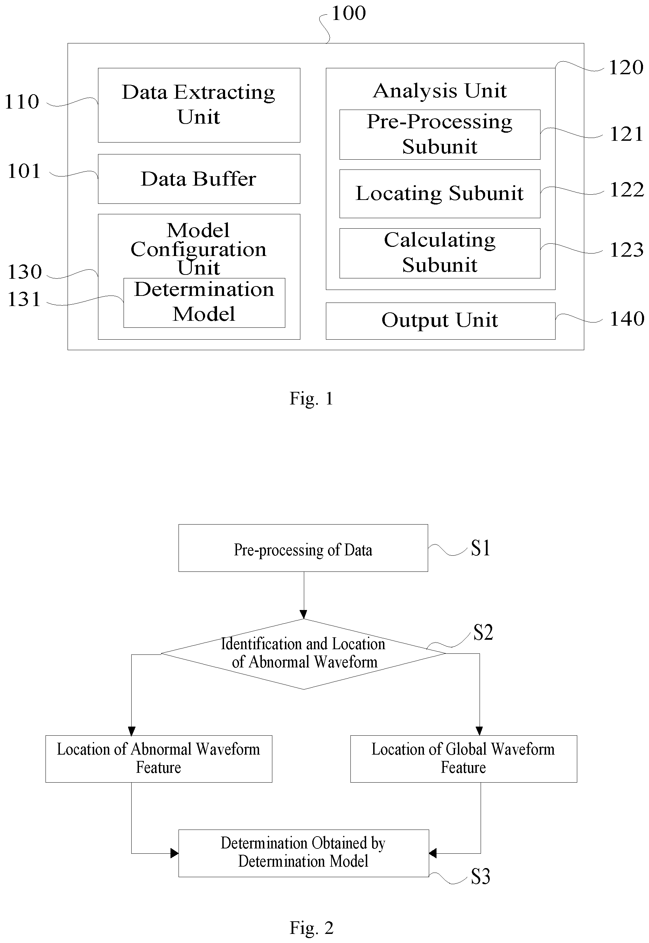

Fig. 1 is a schematic representation of a determination device for determining the origin of an arrhythmia according to embodiments of the present invention (referred to hereinafter as the "determination device"). Fig. 2 is a flow chart illustrating how an analysis unit operates in accordance with an embodiment of the present invention. The determination device according to embodiments of the present invention will be described below in detail with reference to Figs. 1 and 2.

Referring to Fig. 1, in an embodiment of the present invention, the determination device 100 includes: a data extracting unit 110 for extracting ECG data over a predetermined period of time; an analysis unit 120 for processing the ECG data extracted by the data extracting unit 110 and inputting the processed data to a determination model 131, where a calculation is performed thereon to produce origin information about the origin of the arrhythmia; a model configuration unit 130 for configuring the determination model 131; and an output unit 140 for outputting the origin information.

Specifically, in order to extract the ECG data, the data extracting unit 110 may extract, according to a request from a user, an upper-level system or a lower-level system, the ECG data (either real-time or non-real-time) over the predetermined period of time, for example, from an ECG acquisition module or an ECG storage member. For example, the data extracting unit 110 may extract the ECG data over the predetermined period of time from cardiac electrical signals in a multi-lead ECG, such as an 8- Lead ECG, 12- Lead ECG or 20-Lead ECG.

The data extracting unit 110 may extract the ECG data using, for example, a data reading program or device, and the predetermined period of time and other extracting conditions are configurable as desired. For example, a sampling frequency of the data extracting unit 110 and a frequency of ECG data to be extracted can be configured so that, for example, only ECG data in the frequency range from 128 Hz to 10,000 Hz is extracted. In order to reduce the space occupied by the extracted ECG data (e.g., as a data packet) without affecting the integrity of the ECG data, the sampling frequency of the data extracting unit 110 is preferably configured as 512 Hz.

The determination device 100 may include a data buffer 101, in which the ECG data extracted by the data extracting unit 110 is stored. Specifically, the data buffer 101 may be, for example, a random access memory device having a storage capacity required to be not smaller than the size of the data packet of the ECG data extracted by the data extracting unit 110. A minimum capacity of the data buffer 101 can be calculated according to the following equation (1):

$$\text{Minimum Capacity of Data Buffer} = \text{Sampling Frequency} \times 2 \times \text{Period Duration} \times \text{Lead Count} \tag{1}$$

For example, for the ECG data from a 12-Lead ECG, let the sampling frequency be 512 Hz and the predetermined period duration be 10 seconds, then according to Eqn. (1), the minimum capacity of the data buffer will be equal to $512 \times 2 \times 10 \times 12/2^{10} = 120$ KB. That is, it is necessary for the data buffer 101 to have a storage capacity of more than 120 KB.

The analysis unit 120 is adapted to process the ECG data extracted by the data extracting unit 110 and input the extracted data to the determination model 131, where a calculation is performed responsively thereon. Moreover, the analysis unit 120 may include a pre-processing subunit 121, a locating subunit 122 and a calculating subunit 123. Referring to Fig. 2, the analysis unit 120 may perform an analysis on the ECG data extracted by the data extracting unit 110, which may involve the steps of:

S1) pre-processing the ECG data by the pre-processing subunit 121, which may, in particular, include operations such as baseline drift correction and band-pass filtering for eliminating, in the ECG data, electromyographical (EMG) interference, power-line frequency interference, motion artifacts, etc.;

S2) locating an abnormal waveform in the pre-processed ECG data by the locating subunit 122 and acquiring at least one abnormal waveform feature and values of the abnormal waveform feature, the abnormal waveform feature(s) being related to the arrhythmia; and

S3) inputting, by the calculating subunit 123, the values of the abnormal waveform feature(s) to the determination model 131, which then perform calculations to obtain the origin information.

In step S2, the locating subunit 122 may determine the abnormal waveform and/or a global waveform in the ECG data. Here, the term "abnormal waveform" refers to an abnormal heartbeat signal in the ECG data, which embodies features of the arrhythmia, while "global waveform" means a normal heartbeat signal in the ECG data, which embodies features of a normal heart rhythm. Specifically, if the ECG data contains an abnormal heartbeat signal in relation to the arrhythmia, the locating subunit 122 may locate the abnormal waveform and acquire at least one abnormal waveform feature and values of the abnormal waveform feature. Otherwise, if the ECG data does not contain any abnormal heartbeat signal related to the arrhythmia, the locating subunit 122 may locate a global waveform and acquire at least one global waveform feature in relation to the normal heart rhythm and values of the global waveform feature. Alternatively, the values of the abnormal waveform features and the values of the global waveform features may also be acquired from ECG data within a different predetermined period duration.

For example, when the arrhythmia is a ventricular premature beat ("VPB"), the locating subunit 122 may locate a VPB-related abnormal heartbeat signal in the ECG data and acquire one or more abnormal waveform features and values of the abnormal waveform feature. In addition, the locating subunit 122 may also acquire global waveform feature(s) of a normal heart rhythm in the remainder of the ECG data as well as values of the global waveform feature. Those skilled in the art can know how to locate the abnormal and global waveforms and acquire their features from published information, and any further description thereof will be omitted here.

The analysis unit 120 may also include a storage subunit (not shown) for storing the values of the abnormal waveform features and/or the values of the global waveform features, as well as the origin information about the origin that is subsequently obtained from the calculation performed by the determination model 131. Alternatively, the determination device 100 may include a storage unit for storing the values of the abnormal waveform features and/or the values of the global waveform features and the origin information about the origin.

In some embodiments of the present invention, the values of the abnormal waveform feature(s) acquired by the locating subunit 122 may be organized as an abnormal waveform data array with elements corresponding to the respective values of the abnormal waveform feature(s). Likewise, the values of the global waveform feature(s) may be organized as a global waveform data array with elements corresponding to the respective values of the global waveform feature(s). Furthermore, the abnormal waveform data array and/or the global waveform data array may be input by the calculating subunit 123 to, and calculated in, the determination model 131. The abnormal waveform data array and the global waveform data array will be described in greater detail below with the ECG data being extracted from a 12-Lead ECG and the arrhythmia being a VPB as an example.

For ECG data extracted from a 12-Lead ECG, VPB-related abnormal waveform features may include the following six items: an abnormal QRS waveform direction, an abnormal QRS waveform type, an abnormal QRS waveform amplitude, an abnormal QRS waveform duration, an abnormal waveform PR interval and an abnormal waveform QTc interval. Accordingly, the VPB-related abnormal waveform data array may be constructed as a 12-row, 6-column data array A[12][6], where the $1^{st}$ to $12^{th}$ rows in the data array A[12][6] may be defined as corresponding to respective values of the abnormal waveform feature(s) obtained from Lead I, Lead II, Lead III, Lead aVR, Lead aVL, Lead aVF, Lead V1, Lead V2, Lead V3, Lead V4, Lead V5 and Lead V6, and the $1^{st}$ to $6^{th}$ columns in the data array A[12][6] may be defined as corresponding to the values of six aforementioned respective abnormal waveform features. In this way, the elements in the data array A[12][6] correspond to the respective values of the VPB-related abnormal waveform features obtained by the analysis unit 120, and the values of the abnormal waveform features may be obtained in the following manner.

The abnormal QRS waveform direction may be defined as assuming one of the following three values: "1" (representing the deflection of the abnormal QRS waveform upward); "0" (representing that the abnormal QRS waveform segment is isoelectric); and "-1" (representing the deflection of the abnormal QRS waveform downward).

The abnormal QRS waveform type may be any of qR, qRs, Rs, R, rsR, Rr, rs, RS, rSr, Qr, QS and QR, represented respectively by one of the numbers 1 to 12 as shown in Table 1.

Table 1

| Number | 1 | 2 | 3 | 4 | 5 | 6 | 7 | 8 | 9 | 10 | 11 | 12 |
|--------|---|---|---|---|-----|----|----|----|-----|----|----|----|
| Type | qR | qRs | Rs | R | rsR | Rr | rs | RS | rSr | Qr | QS | QR |

The values of the abnormal QRS waveform amplitude, the abnormal QRS waveform duration, the abnormal PR interval and the abnormal QTc interval may be obtained from the ECG data associated with the VPB, either directly or after the data has been processed as desired. In some embodiments, the abnormal QRS waveform amplitude may refer to a QRS waveform amplitude greater than 1.0 mV in Lead V1 (i.e., $R_{V1} > 1.0$ mV), and to a QRS waveform amplitude greater than 2.5 mV in Lead V5 (i.e., $R_{V5} > 2.5$ mV). The abnormal QRS waveform duration may refer to a QRS duration of greater than 0.12 s. The abnormal PR interval may refer to a PR interval greater than 0.2 s. The abnormal QTc interval may refer to a QTc interval greater than 400 ms. However, the present invention is not so limited, and the above various thresholds may also be determined as different values as required.

With this approach, the following abnormal waveform data array A[12][6] may be obtained, with each element thereof corresponding to a respective one of the values of the VPB-related abnormal waveform features that are obtained by the analysis unit 120.

$$\begin{bmatrix} 0 & 7 & 0.5 & 0.12 & 0.32 & 0.40 \\ -1 & 11 & 2 & 0.20 & 0.32 & 0.38 \\ -1 & 11 & 2 & 0.20 & 0.28 & 0.40 \\ 1 & 4 & 1.2 & 0.24 & 0.28 & 0.41 \\ 1 & 4 & 1.2 & 0.24 & 0.24 & 0.40 \\ -1 & 11 & 1.8 & 0.20 & 0.28 & 0.38 \\ -1 & 4 & 1.4 & 0.18 & 0.24 & 0.39 \\ 1 & 4 & 2.5 & 0.20 & 0.25 & 0.40 \\ 1 & 3 & 1.2 & 0.18 & 0.27 & 0.37 \\ 1 & 3 & 1.6 & 0.20 & 0.27 & 0.40 \\ 1 & 8 & 3.5 & 0.20 & 0.28 & 0.40 \\ 0 & 8 & 1.5 & 0.18 & 0.28 & 0.40 \end{bmatrix}$$

Likewise, values of the global waveform feature(s) may be obtained as a global waveform data array. Continuing the example of a 12-Lead ECG, global waveform features may include the following thirteen items: a heart rate, a P-waveform direction, a P-waveform amplitude, a P-waveform duration, a global QRS waveform direction, a global QRS waveform type, a global QRS waveform amplitude, a global QRS waveform duration, a T-waveform direction, a T-waveform amplitude, a T-waveform duration, a global PR interval and a global QTc interval. Accordingly, the normal heart rhythm-related global waveform data array may be constructed as a 12-row, 13-column data array B[12][13], where the 1st to 12th rows in the data array B[12][13] may be defined as corresponding to respective Lead I, Lead II, Lead III, Lead aVR, Lead aVL, Lead aVF, Lead V1, Lead V2, Lead V3, Lead V4, Lead V5 and Lead V6, and the 1st to 13th columns in the data array B[12][13] may be defined as corresponding to values of the thirteen aforementioned respective global waveform features. In this way, the elements in the data array B[12][13] correspond to the respective values of global waveform features obtained by the analysis unit 120, the values of global waveform features may be obtained in the following manner.

The global QRS waveform direction may be defined as assuming one of the following three values: "1" (representing the deflection of the global QRS waveform upward); "0" (representing that the global QRS waveform segment is isoelectric); and "-1" (representing the deflection of the global QRS waveform downward).

With similarity to Table 1, the global QRS waveform type may be one of 12 different global QRS waveform types, each denoted by a respective one of the numbers 1-12.

The values of the heart rate, P-waveform direction, P-waveform amplitude, P-waveform duration, global QRS waveform amplitude, global QRS waveform duration, T-waveform direction, T-waveform amplitude, T-waveform duration, global PR interval and global QTc interval may be obtained from global waveform data using any suitable method known in the art, either directly or after the data has been processed as desired.

With this approach, the following global waveform data array B[12][13] may be obtained, with each element thereof corresponding to a respective one of the values of global waveform features that are obtained by the analysis unit 120.

$$
\begin{bmatrix}
83 & 1 & 0.10 & 90 & 0 & 7 & 0.5 & 0.12 & 1 & 0.20 & 0.20 & 0.32 & 0.40 \\
83 & -1 & 0.15 & 110 & -1 & 11 & 2 & 0.20 & 1 & 1.00 & 0.30 & 0.32 & 0.38 \\
83 & 1 & 0.12 & 120 & -1 & 11 & 2 & 0.20 & 1 & 1.00 & 0.30 & 0.28 & 0.40 \\
83 & 1 & 0.12 & 98 & 1 & 4 & 1.2 & 0.24 & -1 & 0.50 & 0.24 & 0.28 & 0.41 \\
83 & 1 & 0.12 & 96 & 1 & 4 & 1.2 & 0.24 & -1 & 0.50 & 0.30 & 0.24 & 0.40 \\
83 & -1 & 0.15 & 102 & -1 & 11 & 1.8 & 0.20 & 1 & 1.00 & 0.24 & 0.28 & 0.38 \\
83 & 1 & 0.18 & 105 & -1 & 4 & 1.4 & 0.18 & -1 & 0.80 & 0.30 & 0.24 & 0.39 \\
83 & 1 & 0.20 & 98 & 1 & 4 & 2.5 & 0.20 & -1 & 1.40 & 0.30 & 0.25 & 0.40 \\
83 & 1 & 0.10 & 102 & 1 & 3 & 1.2 & 0.18 & 1 & 0.50 & 0.20 & 0.27 & 0.37 \\
83 & 1 & 0.15 & 107 & 1 & 3 & 1.6 & 0.20 & 1 & 0.50 & 0.20 & 0.27 & 0.40 \\
83 & 1 & 0.20 & 99 & 1 & 8 & 3.5 & 0.20 & 1 & 0.50 & 0.20 & 0.28 & 0.40 \\
83 & 1 & 0.15 & 107 & 0 & 8 & 1.5 & 0.18 & 1 & 0.60 & 0.20 & 0.28 & 0.40
\end{bmatrix}
$$

The model configuration unit 130 and the determination model 131 according to embodiments of the present invention will be described below.

The model configuration unit 120 is adapted to configure the determination model 131. In particular, the model configuration unit 120 may be adapted to configure one or more determination models 131.

In some particular embodiments, a plurality of origin categories may be established for various likely arrhythmogenic sites in the heart, and the origin categories embody multiple sites in the heart where the arrhythmia is likely to originate. In other words, the origin of the arrhythmia may include at least one of the origin categories.

In embodiments of the present invention, the plurality of origin categories may include "Left Ventricle", "Inferior Anterior Wall of Right Ventricle", "Inferior Posterior Wall of Right Ventricle", "Bundle of His", "Anterior Part of Free Wall of Right Ventricular Outflow Tract", "Posterior Part of Free Wall of Right Ventricular Outflow Tract", "Middle Part of Free Wall of Right Ventricular Outflow Tract", "Anterior Part of Septal Wall of Right Ventricular Outflow Tract", "Posterior Part of Septal Wall of Right Ventricular Outflow Tract", "Middle Part of Septal Wall of Right Ventricular Outflow Tract" and any others.

In some embodiments, each determination model 131 may include the plurality of origin categories, each being associated with one or more determination indicators, which are used to determine, for the ECG data input to the determination model 131, for example, whether the values of the abnormal waveform features and/or the values of the global waveform features meet corresponding condition. Determinations made on the ECG data input to the determination model 131 based on the one or more determination indicators are helpful in obtaining the origin information about the origin.

The determination model 131 may be, in particular, at least one of a feature classification model, a probability comparison model and a neural network classification model.

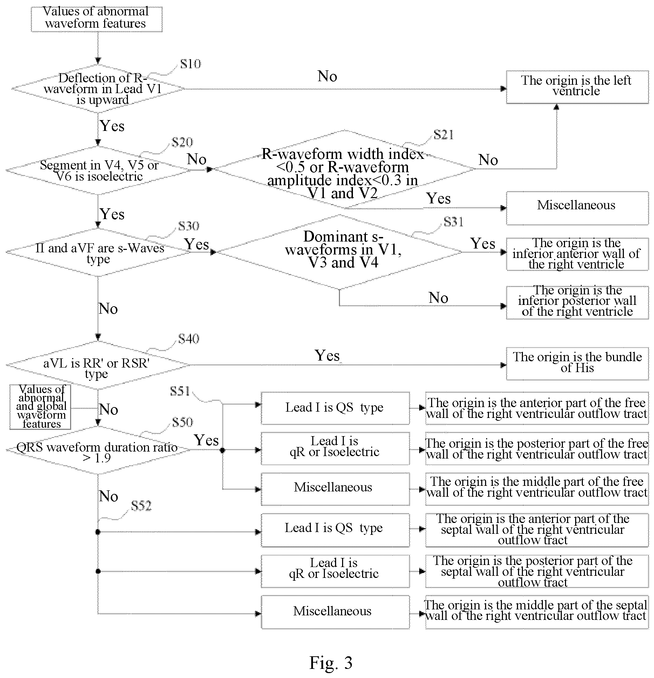

Fig. 3 is a schematic illustration of the feature classification model according to an embodiment of the present invention. The feature classification model is adapted to classify the ECG data input to the determination model based on a plurality of trigger instructions and on a plurality of determination indicators. That is, it is able to classify various ECG data into different origin categories. When the ECG data input to the determination model satisfies all the determination indicators associated with a specific one of the plurality of origin categories, it will be determined that the origin belongs to the specific origin category and that the origin information is information associated with the specific origin category. Here, the term "trigger instructions" correspond to a step (or method) for making determinations by substituting the values of abnormal waveform features into the determination indicators and performing stepwise processing based upon the determination results until the values of abnormal waveform features are classified into one of the origin categories. For example, the trigger instructions may include plurality of instructions for "making a determination for the next determination indicator (or outputting a certain origin category) in the event that the current determination result is Yes" and those for "making a determination for the next determination indicator (or outputting a certain origin category) in the event the current determination result is No".

Continuing with the example of a VPB, the feature classification model according to an embodiment of the present invention using the above-listed origin categories will be described in greater detail below.

Referring to Fig. 3, the feature classification model for determining the VPB's origin includes determination indicators such as "the deflection of the QRS waveform in Lead V1 is upward" and "the abnormal QRS waveform segment in Lead V4, Lead V5 or Lead V6 is isoelectric".

Specifically, the above-discussed abnormal waveform data array A[12][6] and global waveform data array B[12][13] may be input to, and a calculation may be performed thereon in, the feature classification model. Referring to Fig. 3, a method for the feature classification model to obtain origin information about the VPB's origin may include the steps as detailed below.

In step S10, it is determined whether the values of the abnormal waveform features meet the determination indicator of "the deflection of the QRS waveform in Lead V1 is upward". For the abnormal waveform data array A[12][6], this is equivalent to determining whether A[7][1]=1 (i.e., whether the element of the $7^{th}$ row and $1^{st}$ column is equal to 1). If the determination result is Yes, then step S20 is performed. Otherwise, "The origin is left ventricle" is output. That is, the origin of the arrhythmia is determined as belonging to the origin category "Left Ventricle". Accordingly, "The origin is the left ventricle" is output as the origin information.

In step S20, it is determined whether the values of abnormal waveform features meet the determination indicator of "the abnormal QRS waveform segment in Lead V4, Lead V5 or Lead V6 is isoelectric". For the abnormal waveform data array A[12][6], this is equivalent to determining whether A[10][1]=0, or A[11][1]=0, or A[12][1]=0. If the determination is Yes, then step S30 is carried out. Otherwise, step S21 is performed.

In step S21, it is determined whether the values of abnormal waveform features meet the determination indicator of "a QRS waveform width index of <0.5 or a QRS waveform amplitude index of <0.3 in Lead V1 and Lead V2". Here, the term "QRS waveform width index" is defined as a ratio of abnormal QRS waveform durations for the two leads, and the term "QRS waveform amplitude index" is defined as a ratio of abnormal QRS waveform amplitudes for the two leads. For the abnormal waveform data array A[12][6], this is equivalent to determining whether A[7][4]/A[8][4]<0.5 or A[7][4]/A[8][4]<0.3. If the determination is Yes, then "Miscellaneous" is output (according to this embodiment, indicating that the origin category is a category not listed above). Otherwise, "The origin is the left ventricle".

In step S30, it is determined whether the values of abnormal waveform features meet the determination indicator of "s-waveforms in Lead II and Lead aVF". Here, the term "s-waveform" corresponds to the type "rs" or "RS" in Table 1. For the abnormal waveform data array A[12][6], this is equivalent to determining whether A[2][2]=(7 or 8) and A[6][2]=(7 or 8). If the determination is Yes, then step S31 is carried out. Otherwise, step S40 is performed.

In step S31, it is determined whether the values of abnormal waveform features meet the determination indicator of "s-waveforms in Lead V2, Lead V3 and Lead V4". Here, the term "s-waveform" corresponds to the type "rs" or "RS" in Table 1. For the abnormal waveform data array A[12][6], this is equivalent to determining whether A[8][2]=(7 or 8) and A[9][2]=(7 or 8) and A[10][2]=(7 or 8). If the determination is positive, then "The origin is the inferior anterior wall of the right ventricle" is output. Otherwise, "The origin is the inferior posterior wall of the right ventricle" is output.

In step S40, it is determined whether the values of abnormal waveform features meet the determination indicator of "the Lead aVL being R'r or rSR type". For the abnormal waveform data array A[12][6], this is equivalent to determining whether A[4][2]=5 or 6. If the determination is Yes, "The origin is the bundle of His" is output. Otherwise, step S50 is performed.

In step S50, it is determined whether the values of the abnormal and global waveform features meet the determination indicator of "a QRS waveform duration ratio greater than 1.9 in Lead II". For the abnormal waveform data array A[12][6] and the global waveform data array B[12][13], this is equivalent to determining whether A[2][4]/B[2][8]>1.9. If the determination is Yes, step S51 is performed. Otherwise, step S52 is performed.

Step S51 is a sub-step of S50, in which it is determined whether the values of abnormal waveform features meet the determination indicator of "the Lead I being QS type". For the abnormal waveform data array A[12][6], this is equivalent to determining whether A[1][6]=11. If the determination is Yes, "The origin is the anterior part of the free wall of the right ventricular outflow tract" is output. Otherwise, it is further determined whether the values of abnormal waveform features meet the determination indicator of "qR or isoelectric in Lead I". For the abnormal waveform data array A[12][6], this is equivalent to determining whether A[1][2]=1 or 1A[1][1]=0. If the determination is Yes, "The origin is the posterior part of the free wall of the right ventricular outflow tract" is output. Otherwise, "The origin is the middle part of the free wall of the right ventricular outflow tract" is output.

Step S52 is another sub-step of S50, in which it is determined whether the values of abnormal waveform features meet the determination indicator of "QS in Lead I". For the abnormal waveform data array A[12][6], this is equivalent to determining whether A[1][6]=11. If the determination is Yes, "The origin is the anterior part of the septal wall of the right ventricular outflow tract" is output. Otherwise, it is further determined whether the values of abnormal waveform features meet the determination indicator of "qR or isoelectric in Lead I". For the abnormal waveform data array A[12][6], this is equivalent to determining whether A[1][2]=1 or A[1][1]=0. If the determination is Yes, "The origin is the posterior part of the septal wall of the right ventricular outflow tract" is output. Otherwise, "The origin is the middle part of the septal wall of the right ventricular outflow tract" is output.

From the calculation and classification performed on the ECG data input to the feature classification model in steps S10 to S52, the origin information about the origin of the arrhythmia can be obtained.

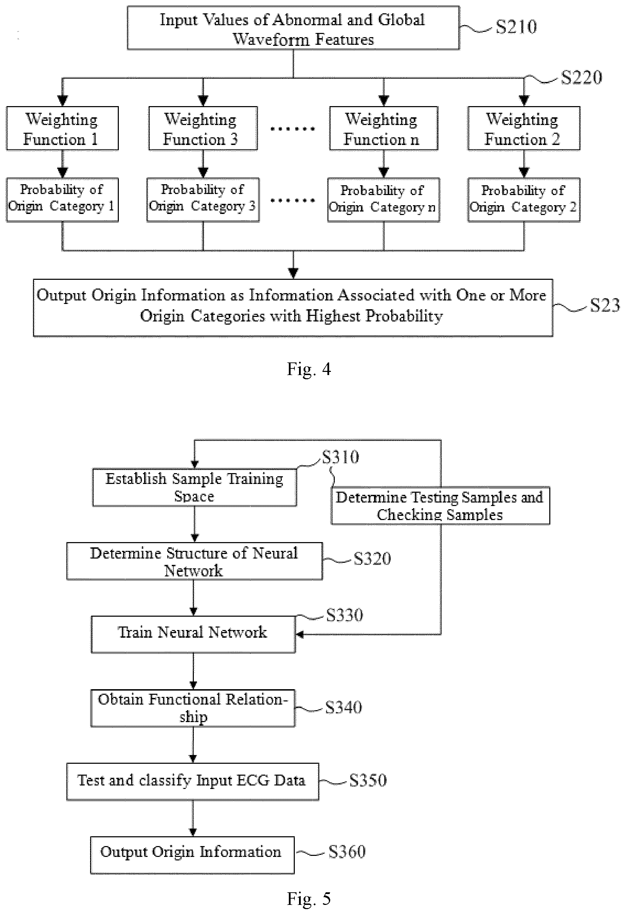

Fig. 4 is a schematic illustration of the probability comparison model according to an embodiment of the present invention, which uses multiple weighting functions for calculating probabilities of the origin of the arrhythmia belonging to the respective origin categories by substituting the ECG data input to the probability comparison model into the weighting functions to obtain one or several of the origin categories with the highest probability, which are considered as the origins, and information associated with the origin categories is output as the origin information. The probability comparison model will be described below in greater detail with reference to Fig. 4.

Specifically, the probability comparison model may include weighting functions 1, 2, 3, ..., n, which correspond to the respective origin categories 1, 2, 3, ..., n, where n is an integer greater than or equal to 1. In other words, each of the weighting functions corresponds to a respective one of the origin categories, and each origin category may be associated with one or more determination indicators which are used to make determinations on the ECG data input to the probability comparison model.

As shown in Eqn. (2), each weighting function may be expressed as the sum of the products of the values of the determination indicators associated with the corresponding origin categories and respective weight coefficients:

$$\text{Weighting Function } n = \text{Weight coefficient } 1 \times \text{Value of Determination Indicator } 1 + \text{Weight coefficient } 2 \times \text{Value of Second Determination Indicator } 2 + ... + \text{Weight coefficient } m \times \text{Value of Determination Indicator } m \tag{2}$$

wherein, the determination indicators 1, 2, ..., m are determination indicators associated with the origin category n corresponding to the weighting function n, and m is an integer greater than or equal to 1.

The value of the determination indicator m may be determined as 1 if the ECG data input to the probability comparison model satisfies the determination indicator m, or as 0 when the input values of abnormal waveform feature(s) do not meet the determination indicator m. However, the present invention is not limited to this method for determining the value of the determination indicator m, any method capable of obtaining, in accordance with a general standard selected by any person skilled in the art, the weighting function n that reflects a probability of the arrhythmia's origin belonging to the origin category n is possible.

Each determination indicator may be multiplied with a weight coefficient (e.g., the weight coefficient 1, ..., m in Eqn. (2)) to reflect the indicator's weight (or contribution) in the corresponding weighting function. Different weight coefficients may be applied to different determination indicators and may be initialized with values set by a physician based on his/her experience, or based on available data. Such weight coefficients can be modified during use.

Methods for setting the weight coefficients may include, for example, quantitative statistics, expert assessment or paired comparisons. Here, the term "quantitative statistics" refers to a method for setting weight coefficients for determination indicators in a weighting function on the basis of the degrees of correlation between the determination indicators and the weighting function, while the term "expert assessment" refers to a method for setting the weights based on knowledge or experience from experts and/or textbooks. In addition, in the method of "paired comparisons", for each of the origin categories, pairwise comparisons are carried out on a plurality of associated determination indicators and each determination indicator is provided with a percentage associated with a probability of the arrhythmia's origin belonging to the specific origin category (initial values of the percentages are, for example, allocated by the physician based on his/her experience), thereby obtaining the weight coefficients for the determination indicators in the corresponding weighting function.

Continuing the example of a VPB, for the weighting function for the origin category "Anterior Part of Free Wall of Right Ventricular Outflow Tract", Table 2 summarizes six exemplary determination indicators for this origin category and respective weight coefficients obtained for them by means of paired comparisons.

According to the determination indicators and their weight coefficients presented in Table 2, the weighting function for the origin category "Anterior Part of Free Wall of Right Ventricular Outflow Tract" can be expressed as (0.22 × Value of Determination Indicator 1 + 0.15 × Value of Determination Indicator 2 + 0.20 × Value of Determination Indicator 3 + 0.17 × Value of Determination Indicator 4 + 0.14 × Value of Determination Indicator 5 + 0.11 × Value of Determination Indicator 6), wherein Determination Indicators 1 to 6 are those in the 2nd to 7th rows of Table 2.

Table 2

| Determination Indicator | Paired Comparison | | | | | | | | | | | | | | | Total | Weight |
|---|---|---|---|---|---|---|---|---|---|---|---|---|---|---|---|---|---|
| Upward QRS Waveform Deflection in Lead V1 | 0.7 | 0.5 | 0.7 | 0.7 | 0.7 | | | | | | | | | | | 3.3 | 0.22 |
| Isoelectric QRS waveform segment in Lead V4, V5 or V6 | 0.3 | | | | | 0.3 | 0.5 | 0.5 | 0.7 | | | | | | | 2.3 | 0.15 |
| No s-waveform in Leads II and aVF | | 0.5 | | | | 0.7 | | | | 0.5 | 0.6 | 0.7 | | | | 3 | 0.20 |
| No R'r or rSR in Lead aVL | | | 0.3 | | | | 0.5 | | | 0.5 | | | 0.7 | 0.6 | | 2.6 | 0.17 |
| QRS duration ratio value >1.9 in Lead II | | | | 0.3 | | | | 0.5 | | | 0.4 | | 0.3 | | 0.6 | 2.1 | 0.14 |
| QS type in Lead I | | | | | 0.3 | | | | 0.3 | | | 0.3 | | 0.4 | 0.4 | 1.7 | 0.11 |
| Total | 1 | 1 | 1 | 1 | 1 | 1 | 1 | 1 | 1 | 1 | 1 | 1 | 1 | 1 | 1 | 15 | 1.00 |

In the method of paired comparisons, for each origin category, it is preferred that each weighting function involves five or more determination indicators, more preferably not less than ten determination indicators. Moreover, for each origin category, when the percentages for each pair of determination indicators are to be allocated based on physician experience, for each determination indicator, the weight coefficient may be determined as the average of candidate weight coefficients according to allocation schemes of at least five physicians, in order to ensure high accuracy of the resulting weights. It is to be noted that the determination indicators are not limited to the VPB-related ones provided in the foregoing example, and may also include those chosen based on textbooks or ECG experts' experience.

In the similar way, weighting functions for all the origin categories related to the arrhythmia can be obtained.

Referring to Fig. 4, in some embodiments using the probability comparison model, step S210 is first performed, in which values of the abnormal and global waveform features are input to the probability comparison model (e.g., in the form of the above-discussed abnormal waveform data array and global waveform data array). Step S220 is then performed, in which the ECG data input to the probability comparison model is substituted into the weighting functions for the respective origin categories to calculate the probabilities of the arrhythmia's origin belonging to the respective origin categories. Next, step S230 is performed, in which the resulting probabilities are sorted by their magnitude, and one or several highest probabilities are obtained. Subsequently, the one or several of the origin categories with the highest probabilities are considered as the origins, and information associated with these origin categories is output as the origin information. For each origin category, the higher the corresponding probability is, the more likely it is for the origin of the arrhythmia to belong to the origin category. In the case of the output origin information being in relation to two or more origin categories, a further comparison or further choices may be made between them by the physician.

Fig. 5 is a schematic illustration of the neural network classification model according to an embodiment of the present invention. The neural network classification model utilizes a neural network, which is trained to obtain a functional relationship of a set of ECG data to a set of origin categories. The neural network is adapted to test and classify the ECG data input to the neural network classification model, and the neural network classification model can find, from the classification, the origin category to which the origin of the arrhythmia belongs and output the information associated with the origin category identified from the classification as origin information. The neural network classification model will be described below in greater detail with reference to Fig. 5.

A specific method for obtaining the origin information by the neural network classification model may include the steps as detailed below.

In step S310, a sample training space is established, and testing samples and checking samples are determined.

Training samples in the sample training space may be provided by the physician or chosen from a data storage device. The training samples preferably include ECG data with determination indicators and origin categories. For example, for each origin category, 100 or more 12-Lead ECGs can be provided, and the analysis unit 120 may then locate abnormal waveform feature(s) and/or global waveform feature(s) in each ECG, obtaining a set of values of abnormal waveform feature(s) and/or global waveform feature(s), denoted at $X=\{x_i|i=1, 2, 3, ......\}$, where $x_i$ represents the value of i-th abnormal and/or global waveform feature, and $i\geq100$. Each $x_i$ may correspond to a respective origin category, denoted at $y_i$. In this way, the set X can be mapped to an origin category set $Y=\{y_i|i=1, 2, 3, ......\}$, as indicated by a mapping function $y=r(x)$. Thus, a sample training space with the X-to-Y mapping can be obtained.

In addition, step S310 may also include setting testing samples, i.e., a checking sample space used for checking, as X', and setting checking samples, i.e., a corresponding expected output checking sample space, as Y'.

In step S320, a structure of the neural network is determined. The structure of the neural network may include an input layer (corresponding to the ECG data set X input to the neural network classification model), an output layer (corresponding to the origin category set Y) and a hidden layer, which includes the X-to-Y mapping function, for example.

Step S320 may also include determining a neuron activation function, for example, as the following logistic function of Eqn. (3):

$$\sigma(Z) = \frac{1}{1+e^z} \qquad (3)$$

In some other embodiments, the neuron activation function may be a different function such as a threshold function.

In step S330, the neural network is trained. That is, data training is performed. Training and checking may be performed using the sample training space, the testing samples X' and the corresponding expected output checking sample space Y' until an accuracy rate is tested to be, for example, over 98%.

In step S340, the functional relationship is obtained. From the above steps, the neural network may be defined by a weighting term w and a biasing term b as $y=r(x; w, b)$ and used to classify the origin categories.

In step S350, the ECG data is input, tested and classified. Specifically, the analysis unit 120 may input the values of abnormal and/or global waveform feature(s) to the neural network, so that they are tested and classified.

12

In step S360, the origin information, i.e., information associated with the origin category identified from the classification, is output.

Therefore, the origin category to which the origin of the arrhythmia belongs and the origin information about the origin can be obtained from the processing of the extracted ECG data by the analysis unit 120 and the calculation performed by the determination model 131 on the processed ECG data.

The output unit 140 may output the origin information obtained by the analysis unit 120 to a terminal, which may be a computer system with a display module. Additionally, the determination device 100 may be coupled to a medical record server, which may be another terminal coupled to the output of the output unit 140. In this way, the origin information may be output to the medical record server.

The origin information about the origin of the arrhythmia may be displayed in one or more of the forms of text, images and voice. In some embodiments, a heart model may be displayed on the terminal, and the origin information may be represented as an arrow or color block marked on the heart model. The heart model is preferably a three-dimensional (3D) heart model, in particular, a standard heart model, a modeled heart model or a CT-matched heart model. Here, the term "standard heart model" refers to a heart model which is provided by another application of the terminal and capable of displaying arrhythmogenic sites of different types in the heart, and "modeled heart model" to a heart model obtaining from modeling of the entire heart or part of the heart based on available information. In addition, the term "CT-matched heart model" refers to a 3D heart model obtained by registering a CT image of a patient's heart with a standard or model heart model.

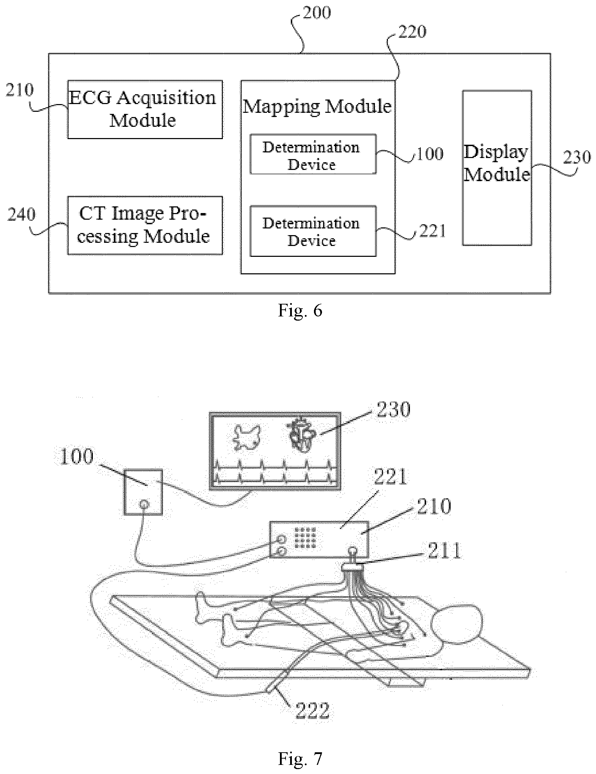

In embodiments of the present invention, there is also provided a mapping system. Fig. 6 is a schematic representation of the mapping system according to an embodiment of the present invention, and Fig. 7 schematically illustrates a mapping process performed by the mapping system according to an embodiment of the present invention. Referring to Figs. 6 and 7, the mapping system 200 includes: an ECG acquisition module 210 for capturing a patient's ECG data; and a mapping module 220 for determining the origin of an arrhythmia and mapping the patient's heart based on origin information about the origin, wherein the mapping module includes the determination device 100 as defined above.

The ECG acquisition module 210 is configured to capture the ECG data from the patient's body surface, for example, using an 8- Lead, 12 Lead - or 20-Lead ECG system. With ECG leads 211 placed on the patient's body surface, the ECG acquisition module 210 is able to detect the patient's cardiac electrical activity in real time.

The mapping module 220 may further include a locating device 221 for locating a site to be mapped. Before the mapping, the determination device 100 may be used to determine the origin of the arrhythmia and provide the origin information indicative of the arrhythmia's origin, based on which, the physician may control or instruct the locating device 221, so that a mapping catheter 222 is delivered to a region of the heart encompassing the origin to conduct focused mapping thereof. In this way, the time required for mapping can be reduced.

The mapping system 200 may further include a display module 230, the display module 230 is capable of displaying both the information about the origin of the arrhythmia and progress in the mapping process.

The mapping system 200 may further include a CT image processing module 240 for establishing a CT-matched heart model by identifying a CT image of the patient's heart and registering it with a standard or modeled heart model.

In summary, the determination device according to embodiments of the present invention is capable of providing a physician with information about the origin of an arrhythmia in a timely manner before or during a procedure, thus allowing direct focused mapping of a cardiac site corresponding to the origin indicated by the origin information. This can reduce the time required for mapping and result in an increase in surgical efficiency. Moreover, the determination device can determine the origin using a general determination model, which is conducive to the standardization and promotion of cardiac mapping technology.

According to the present invention, the mapping system may be a 3D mapping system, and the mapping module includes the determination device for determining the origin of an arrhythmia. Origin information about the origin of the arrhythmia can be obtained by processing ECG data extracted over a predetermined period of time and performing a calculation on the processed data in the determination model, and can be output to help a physician know a cardiac site for focus mapping. Again, this can reduce the time required for mapping and result in improved surgical efficiency.

The description presented above is merely that of a few preferred embodiments of the present invention and does not limit the scope thereof in any sense. Any person skilled in the art can make possible changes and modifications to the subject matter of the present invention based on the above teachings without departing from the spirit and scope of the present invention. Accordingly, any and all simple changes, equivalent alternatives and modifications made to the foregoing embodiments based on the essence of the present invention without departing the scope of the invention fall within the scope thereof.

**Claims**

1. A determination device for an origin of an arrhythmia, configured to determine the origin of the arrhythmia, the determination device comprising:

a data extracting unit configured to extract electrocardiogram (ECG) data over a predetermined period of time;
an analysis unit configured to process the ECG data and input the processed ECG data into a determination model to perform a calculation to obtain an origin information about the origin of the arrhythmia;
a model configuration unit configured to configure the determination model; and
an output unit configured to output the origin information.

2. The determination device of claim 1, wherein the analysis unit comprises:

a pre-processing subunit configured to pre-process the ECG data;
a locating subunit configured to derive at least one abnormal waveform feature and a value of the abnormal waveform feature from the pre-processed ECG data, the abnormal waveform feature being related to the arrhythmia; and
a calculating subunit configured to input the value of the abnormal waveform feature into the determination model to perform a calculation.

3. The determination device of claim 2, wherein the locating subunit is further configured to derive at least one global waveform feature and a value of the global waveform feature from the pre-processed ECG data, the global waveform feature being related to a normal heart rhythm, and wherein the calculating subunit is further configured to input the value of the global waveform feature into the determination model to perform a calculation.

4. The determination device of claim 3, wherein the global waveform feature comprises a heart rate, a P-waveform direction, a P-waveform amplitude, a P-waveform duration, a global QRS waveform direction, a global QRS waveform type, a global QRS waveform amplitude, a global QRS waveform duration, a T-waveform direction, a T-waveform amplitude, a T-waveform duration, a global PR interval and a global QTc interval.

5. The determination device of claim 4, wherein the value of the global waveform feature is presented by a global waveform data array.

6. The determination device of claim 5, wherein each column of the global waveform data array corresponds to values of one item of the global waveform feature.

7. The determination device of claim 6, wherein the ECG data is extracted from an 8-Lead ECG, 12-Lead ECG or 20-Lead ECG, and wherein each row of the global waveform data array corresponds to a respective lead.

9. The determination device of claim 2, wherein the calculating subunit is configured to input an abnormal waveform data array into the determination model, the abnormal waveform data array having elements corresponding to the respective values of the abnormal waveform feature.

10. The determination device of claim 9, wherein the abnormal waveform feature comprises an abnormal QRS waveform direction, an abnormal QRS waveform type, an abnormal QRS waveform amplitude, an abnormal QRS waveform duration, an abnormal PR interval and an abnormal QTc interval.

11. The determination device of claim 10, wherein each column of the abnormal waveform data array corresponds to values of one item of the abnormal waveform feature.

12. The determination device of claim 11, wherein the ECG data is extracted from an 8-Lead ECG, 12-Lead ECG or 20-Lead ECG, and wherein each row of the abnormal waveform data array corresponds to a respective lead.

13. The determination device of any one of claims 1 to 12, wherein the determination model comprises a plurality of origin categories, each associated with one or more determination indicators for making determinations on the ECG data that is input into the determination model, and wherein the origin belongs to at least one of the plurality of origin categories.

**14.** The determination device of claim 13, wherein the plurality of origin categories include "Left Ventricle", "Inferior Anterior Wall of Right Ventricle", "Inferior Posterior Wall of Right Ventricle", "Bundle of His", "Anterior Part of Free Wall of Right Ventricular Outflow Tract", "Posterior Part of Free Wall of Right Ventricular Outflow Tract", "Middle Part of Free Wall of Right Ventricular Outflow Tract" , "Anterior Part of Septal Wall of Right Ventricular Outflow Tract", "Posterior Part of Septal Wall of Right Ventricular Outflow Tract" and "Middle Part of Septal Wall of Right Ventricular Outflow Tract".

**15.** The determination device of claim 13, wherein the determination model is a feature classification model configured to classify the ECG data that is input into the determination model using a plurality of trigger instructions and the determination indicators, and wherein when the ECG data that is input into the feature classification model satisfies all the determination indicators associated with a specific origin category of the plurality of origin categories, the origin is determined as belonging to the specific origin category and the origin information is output as an information associated with the specific origin category.

**16.** The determination device of claim 13, wherein the determination model is a probability comparison model configured with a plurality of weighting functions representing probabilities of the arrhythmia for each of the plurality of origin categories, wherein a calculation is performed by substituting the ECG data input to the probability comparison model into the plurality of weighting functions to obtain one or more of the plurality of origin categories with a highest probability, and wherein the origin is determined as belonging to the one or more of the plurality of origin categories with the highest probability, and the origin information is output as information associated with the one or more of the plurality of origin categories with the highest probability.

**17.** The determination device of claim 16, wherein each of the plurality of weighting functions comprises the sum of the products of values of the determination indicators associated with the plurality of origin categories and respective weight coefficients, each of the weight coefficients reflecting a weight of the multiplied value of the determination indicator in the plurality of weighting functions.

**18.** The determination device of claim 17, wherein the weight coefficients are obtained from paired comparisons.

**19.** The determination device of claim 13, wherein the determination model is a neural network classification model configured to train a neural network to derive a functional relationship between a set of ECG data and a set of the plurality of origin categories and to test and classify the ECG data input to the neural network classification model, and wherein the origin belongs to one of the plurality of origin categories identified from the classification and the origin information is output as an information associated with the origin category identified from the classification.

**20.** The determination device of claim 1, wherein the output unit is configured to output the origin information to a terminal, and the origin information is marked on a heart model displayed on the terminal.

**21.** The determination device of claim 20, wherein the heart model is a standard heart model, a modeled heart model or a CT-matched heart model.

**22.** The determination device of claim 1, wherein the arrhythmia is a ventricular premature beat.

**23.** A mapping system for mapping a patient's heart, the mapping system comprising:

an electrocardiogram (ECG) acquisition module for capturing ECG data of the patient; and
a mapping module for determining an origin of an arrhythmia and mapping the patient's heart,
wherein the mapping module comprises the determination device of any one of claims 1 to 22.

**24.** The mapping system of claim 23, further comprising:

a display module for displaying an origin information about the origin; and/or
a CT image processing module for identifying a CT image of the patient's heart and registering the CT image with a standard or modeled heart model to establish a CT-matched heart model.

100

Fig. 1

Fig. 2

Values of abnormal
waveform features

S10

Deflection of R-
waveform in Lead V1
is upward

No → The origin is the left ventricle

Yes

S20

Segment in V4, V5 or V6 is isoelectric

No → S21 R-waveform width index <0.5 or R-waveform amplitude index<0.3 in V1 and V2

No → The origin is the left ventricle

Yes → Miscellaneous

Yes

S30

II and aVF are s-Waves type

Yes → S31 Dominant s-waveforms in V1, V3 and V4

Yes → The origin is the inferior anterior wall of the right ventricle

No → The origin is the inferior posterior wall of the right ventricle

No

S40

aVL is RR' or RSR' type

Yes → The origin is the bundle of His

Values of abnormal and global waveform features

No

S51

S50

QRS waveform duration ratio > 1.9

Yes

Lead I is QS type → The origin is the anterior part of the free wall of the right ventricular outflow tract

Lead I is qR or Isoelectric → The origin is the posterior part of the free wall of the right ventricular outflow tract

Miscellaneous → The origin is the middle part of the free wall of the right ventricular outflow tract

No   S52

Lead I is QS type → The origin is the anterior part of the septal wall of the right ventricular outflow tract

Lead I is qR or Isoelectric → The origin is the posterior part of the septal wall of the right ventricular outflow tract

Miscellaneous → The origin is the middle part of the septal wall of the right ventricular outflow tract

Fig. 3

Input Values of Abnormal and Global Waveform Features ⟶ S210

S220

| Weighting Function 1 | Weighting Function 3 | ...... | Weighting Function n | Weighting Function 2 |

| Probability of Origin Category 1 | Probability of Origin Category 3 | ...... | Probability of Origin Category n | Probability of Origin Category 2 |

Output Origin Information as Information Associated with One or More Origin Categories with Highest Probability ⟶ S23

Fig. 4

Establish Sample Training Space — S310

Determine Testing Samples and Checking Samples

Determine Structure of Neural Network — S320

Train Neural Network — S330

Obtain Functional Relation-ship — S340

Test and classify Input ECG Data — S350

Output Origin Information — S360

Fig. 5

18

Fig. 6

Fig. 7

## INTERNATIONAL SEARCH REPORT

| International application No. |
|---|
| **PCT/CN2019/093836** |

**A. CLASSIFICATION OF SUBJECT MATTER**

A61B 5/0452(2006.01)i;  A61B 5/0402(2006.01)i

According to International Patent Classification (IPC) or to both national classification and IPC

**B. FIELDS SEARCHED**

Minimum documentation searched (classification system followed by classification symbols)

A61B; A61N

Documentation searched other than minimum documentation to the extent that such documents are included in the fields searched

Electronic data base consulted during the international search (name of data base and, where practicable, search terms used)

CNKI CNABS CNTXT WPI EPODOC: 上海微创电生理, 夏云龙, 张清淳, 沈刘娉, 王路, 孙毅勇, 心律失常, 心律异常, 心脏异常, 房颤, 室颤, 早搏, 起源, 定位, 位置, 区域, 组织, 分类, 模型, 神经网络, cardiac, heart, arrhythmia, abnormal, wrong, atrial, fibrillation, premature, beat+, ventricular, orgin+, posit+, location, region, tissue, classif+, model

**C. DOCUMENTS CONSIDERED TO BE RELEVANT**

| Category* | Citation of document, with indication, where appropriate, of the relevant passages | Relevant to claim No. |
|---|---|---|
| PX | CN 109091138 A (SHANGHAI MICROPORT EP MEDTECH CO., LTD.) 28 December 2018 (2018-12-28) <br> description, paragraphs [0043]-[0077], claims 1-17, and figures 1-7 | 1-7, 9-24 |
| X | CN 102579034 A (LU, CAIYI) 18 July 2012 (2012-07-18) <br> description, paragraphs [0040]-[0054], and figure 1 | 1, 13, 22 |
| Y | CN 102579034 A (LU, CAIYI) 18 July 2012 (2012-07-18) <br> description, paragraphs [0040]-[0054], and figure 1 | 2-7, 9-12, 14-21, 23-24 |
| Y | CN 103202727 A (GENERAL ELECTRIC CO.) 17 July 2013 (2013-07-17) <br> description, paragraphs [0029]-[0032] and [0037]-[0043], and figures 1 and 2 | 2-7, 9-12, 14-21, 23-24 |
| A | CN 106073763 A (THE REGENTS OF THE UNIVERSITY OF CALIFORNIA ET AL.) 09 November 2016 (2016-11-09) <br> entire document | 1-7, 9-24 |
| A | WO 02087695 A1 (MEDTRONIC, INC.) 07 November 2002 (2002-11-07) <br> entire document | 1-7, 9-24 |

☑ Further documents are listed in the continuation of Box C. ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **06 September 2019** | **29 September 2019** |

| Name and mailing address of the ISA/CN | Authorized officer |
|---|---|
| **China National Intellectual Property Administration** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2019/093836** |

### C. DOCUMENTS CONSIDERED TO BE RELEVANT

| Category* | Citation of document, with indication, where appropriate, of the relevant passages | Relevant to claim No. |
|---|---|---|
| A | CN 101254096 A (SIEMENS MEDICAL SOLUTIONS USA, INC.) 03 September 2008 (2008-09-03)<br>entire document | 1-7, 9-24 |
| A | US 2007219452 A1 (COHEN, R.J. ET AL.) 20 September 2007 (2007-09-20)<br>entire document | 1-7, 9-24 |

Form PCT/ISA/210 (second sheet) (January 2015)

**INTERNATIONAL SEARCH REPORT**
Information on patent family members

| International application No. |
| --- |
| **PCT/CN2019/093836** |

| Patent document cited in search report | | | Publication date (day/month/year) | Patent family member(s) | | | Publication date (day/month/year) |
| --- | --- | --- | --- | --- | --- | --- | --- |
| CN | 109091138 | A | 28 December 2018 | None | | | |
| CN | 102579034 | A | 18 July 2012 | CN | 102579034 | B | 11 December 2013 |
| CN | 103202727 | A | 17 July 2013 | CN | 103202727 | B | 25 November 2015 |
| | | | | US | 2013184697 | A1 | 18 July 2013 |
| CN | 106073763 | A | 09 November 2016 | US | 10058262 | B2 | 28 August 2018 |
| | | | | US | 2017340229 | A1 | 30 November 2017 |
| | | | | RU | 2014127197 | A | 10 February 2016 |
| | | | | US | 9392948 | B2 | 19 July 2016 |
| | | | | CA | 2858575 | A1 | 13 June 2013 |
| | | | | CN | 104254277 | B | 03 May 2017 |
| | | | | AU | 2012347477 | A1 | 24 July 2014 |
| | | | | IL | 233020 | D0 | 31 July 2014 |
| | | | | CN | 104254277 | A | 31 December 2014 |
| | | | | AU | 2012347478 | A1 | 24 July 2014 |
| | | | | IN | 1383MUN2014 | A | 03 April 2015 |
| | | | | JP | 2015505695 | A | 26 February 2015 |
| | | | | US | 9724009 | B2 | 08 August 2017 |
| | | | | US | 2013150740 | A1 | 13 June 2013 |
| | | | | EP | 2787879 | A1 | 15 October 2014 |
| | | | | IN | 1385MUN2014 | A | 03 April 2015 |
| | | | | BR | 112014013831 | A2 | 13 June 2017 |
| | | | | US | 2015038861 | A1 | 05 February 2015 |
| | | | | MX | 2014006904 | A | 16 November 2015 |
| | | | | US | 9408536 | B2 | 09 August 2016 |
| | | | | RU | 2014127196 | A | 10 February 2016 |
| | | | | EP | 2787880 | A1 | 15 October 2014 |
| | | | | US | 2013150742 | A1 | 13 June 2013 |
| | | | | WO | 2013086468 | A1 | 13 June 2013 |
| | | | | WO | 2013086469 | A1 | 13 June 2013 |
| | | | | US | 2016324434 | A1 | 10 November 2016 |
| | | | | JP | 6408911 | B2 | 17 October 2018 |
| | | | | BR | 112014013834 | A2 | 13 June 2017 |
| | | | | KR | 20140114361 | A | 26 September 2014 |
| | | | | CN | 104185443 | B | 06 July 2016 |
| | | | | JP | 2015504701 | A | 16 February 2015 |
| | | | | CN | 104185443 | A | 03 December 2014 |
| | | | | MX | 2014006801 | A | 03 March 2015 |
| | | | | KR | 20140113942 | A | 25 September 2014 |
| | | | | CA | 2858604 | A1 | 13 June 2013 |
| | | | | IL | 233019 | D0 | 31 July 2014 |
| WO | 02087695 | A1 | 07 November 2002 | US | 7058443 | B2 | 06 June 2006 |
| | | | | US | 2002183636 | A1 | 05 December 2002 |
| CN | 101254096 | A | 03 September 2008 | CN | 101254096 | B | 24 April 2013 |
| | | | | US | 7715907 | B2 | 11 May 2010 |
| | | | | US | 2008214945 | A1 | 04 September 2008 |
| US | 2007219452 | A1 | 20 September 2007 | WO | 2007109123 | A2 | 27 September 2007 |
| | | | | US | 7792563 | B2 | 07 September 2010 |

Form PCT/ISA/210 (patent family annex) (January 2015)